# EUROPEAN PATENT APPLICATION

(11) **EP 0 955 284 A1**
(43) Date of publication of application: **10.11.1999**
(21) Application number: 99303177.2
(22) Date of filing: 23.04.1999
(51) Int. Cl.: C07C 29/04

(54) **Olefin hydration process**

(30) Priority: 29.04.1998 GB 9809210
(71) Applicant: BP Chemicals Limited, London EC2M 7BA (GB)
(72) Inventor: Haining, Gordon John, BP Chemicals Limited, Grangemouth, Stirlingshire, FK3 9XH (GB); Smith, Mark Royston, BP Chemicals Limited, Grangemouth, Stirlingshire, FK3 9XH (GB); Turner, Malcolm John, BP Chemicals Limited, Sunbury-on-Thames, Middlesex TW16 7LN (GB)
(74) Representative: Smith, Julian Philip Howard

(57) **Abstract**

This invention relates to a process for co-producing ethanol and isopropanol by hydrating a mixture of ethylene and propylene in the vapour phase in the presence of a catalyst composition comprising a heteropolyacid catalyst supported on a siliceous support. The process is characterised in that the hydration reaction of the mixed olefin feed is carried out under specific reaction conditions.

## Description

The present invention relates to a process for the hydration of olefins to alcohols and especially to the co-production of ethanol and isopropanol by said process.

It is well known to support acid catalysts such as eg phosphoric acid or heteropolyacids on a carrier such as eg silica or clay and to use such a supported catalyst for the hydration of olefins such as ethylene or propylene in the vapour phase to the corresponding alcohols. Numerous prior art publications describe such a procedure including those disclosed in GB-A-1570650, US-A-4808559, GB-A-1371905, US-A-4038211, US-A-4012452, GB-A-1476534, GB-A-1306141, US-A-3996338 and CAN-A-844004. However, all ofthese processes only describe the production of a single alcohol from a relatively pure olefin feed such as eg ethylene to ethanol and propylene to isopropanol. As has been described previously, eg in our own prior published EP-A-578441, it has always been believed that the reaction conditions for making ethanol from ethylene are different from those needed to produce iso-propanol from propylene and hence the two alcohols could not be co-produced. EP-A-578441 for instance states that to produce ethanol from ethylene the catalyst is preferably heated from 230-260°C and the ethylene/water mixture is passed through the catalyst bed at a preferred space velocity of 0.02-0.05 g/min/cm³ and a reaction pressure of 3000-10000 KPa. In contrast, for making isopropanol from propylene the same catalyst is preferably heated at a temperature in the range from 185-205°C and the propylene/water mixture is passed through the catalyst bed at a preferred space velocity of 0.02-0.07 and a reaction pressure of 2000-7600 KPa. Such use of distinctive process parameters to produce each of these alcohols not only applies to phosphoric acid catalyst but also applies to other catalysts such as heteropolyacids. The conclusion from all of this is that the process conditions used for making ethanol and isopropanol by hydration of the corresponding olefin are significantly different and that these cannot be co-produced in commercially viable yields. However, it is desirable to co-produce these alcohols in a single process due to the commercial sources of the olefinic feedstock where ethylene and propylene are usually admixed to a degree and such feedstock may be used as such without incurring the penalty of costs involved in the separation of these olefins prior to being used in the hydration reaction.

Attempts to co-produce these alcohols from a mixed olefin feedstock have hitherto failed because raising the reaction temperature increases the formation of ethanol but substantially decreases the formation of isopropanol. Although a 3 : 1 ratio of isopropanol : ethanol can be achieved, the temperature required for this distribution is such that the production of both is well away from the optimum thereby resulting in a very low absolute productivity of both alcohols. Similarly, attempts to increase the production of one of these alcohols by altering the reaction parameters, such as eg mole ratios of olefin to water; the concentration of each olefin in the mixed olefins feed; or the reaction partial pressures; always suppressed the production of the other alcohol.

It has now been found that by using optimised conditions and a supported phosphoric acid catalyst both ethanol and isopropanol can be co-produced in commercially viable yields using a mixed olefin feedstock.

Accordingly the present invention is a process for co-producing ethanol and isopropanol by hydrating a mixture of ethylene and propylene in the vapour phase in the presence of a catalyst composition comprising a heteropolyacid catalyst supported on a siliceous support, characterised in that the hydration reaction of the mixed olefin feed is carried out under the following conditions:

| **Parameter** | **Ranges** | |
|---|---|---|
| | **General** | **Preferred** |
| Reaction Temp (°C) | 140-270 | 170-250 |
| Reaction Pressure (KPa) | 1000-9000 | 2000-7000 |
| C₂H₄:C₃H₆ mole ratio | 0.3-4.0 | 1.0-3.0 |
| Olefin : Water mole ratio | 0.2-1.5 | 0.3-0.6 |
| GHSV-Olefin/Water mix | 750-5000 | 1000-2000 |
| HPA Loading (g/l)* | 50-200 | 60-150 |

| | | |
|---|---|---|
| *on the silica support | | |

The term "heteropolyacids" as used herein and throughout the specification is meant to include the free acids (hereafter "HPA") and neutral and acidic salts thereof.
By acidic salts is meant salts of the HPAs in which some of the hydrogen ions are replaced by basic cations such as eg alkali metal cations. The heteropolyacids used to prepare the olefin hydration catalysts of the present invention therefore include the free acids and the coordination-type salts thereof in which the anion is a complex, high molecular weight entity. Typically, the anion is comprises 2-18 oxygen-linked polyvalent metal atoms, which are called peripheral atoms. These peripheral atoms surround one or more central atoms in a symmetrical manner. The peripheral atoms are usually one or more of molybdenum, tungsten, vanadium, niobium, tantalum and other metals. The central atoms are usually silicon or phosphorus but can comprise any one of a large variety of atoms from Groups I-VIII in the Periodic Table of elements. These include, for instance, cupric ions; divalent beryllium, zinc, cobalt or nickel ions; trivalent boron, aluminium, gallium, iron, cerium, arsenic, antimony, phosphorus, bismuth, chromium or rhodium ions; tetravalent silicon, germanium, tin, titanium, zirconium, vanadium, sulphur, tellurium, manganese nickel, platinum, thorium, hafnium, cerium ions and other rare earth ions; pentavalent phosphorus, arsenic, vanadium, antimony ions; hexavalent tellurium ions; and heptavalent iodine ions. Such heteropolyacids are also known as "polyoxoanions", "polyoxometallates" or "metal oxide clusters". The structures of some of the well known anions are named after the original researchers in this field and are known eg as Keggin, Wells-Dawson and Anderson-Evans-Perloff structures.

Heteropolyacids usually have a high molecular weight eg in the range from 700-8500 and include dimeric complexes. They have a relatively high solubility in polar solvents such as water or other oxygenated solvents, especially if they are free acids and in the case of several salts, and their solubility can be controlled by choosing the appropriate counterions. Specific examples of heteropolyacids that may be used as the catalysts in the present invention include:

| | |
|---|---|
| 12-tungstophosphoric acid | H₃[PW₁₂O₄₀].xH₂0 |
| 12-molybdophosphoric acid | H₃[PMo₁₂O₄₀].xH₂O |
| 12-tungstosilicic acid | H₄[SiW₁₂O₄₀].xH₂O |
| 12-monosodium tungstosilicic acid | H₃Na[SiW₁₂O₄₀].xH₂O |
| 12-molybdosilicic acid | H₄[SiMo₁₂O₄₀].xH₂O |
| Potassium tungstophosphate | K₆[P₂W₁₈O₆₂].xH₂O |
| Sodium molybdophosphate | Na₃[PMo₁₂O₄₀].xH₂O |
| Ammonium molybdodiphosphate | (NH₄)₆[P₂Mo₁₈O₆₂].xH₂O |
| Sodium tungstonickelate | Na₄[NiW₆O₂₄H₆].xH₂O |
| Ammonium molybdodicobaltate | (NH₄)[Co₂Mo₁₀O₃₆].xH₂O |
| Cesium hydrogen tungstosilicate | Cs₃H[Siw₁₂O₄₀].xH₂O |
| Potassium molybdodivanado phosphate | K₅[PMoV₂O₄₀].xH₂O |

It should be noted that the polyvalent oxidation states and hydration states of the heteropolyacids as stated previously and as represented in the typical formulae of some specific compounds only apply to the fresh acid before it is impregnated onto the support, and especially before it is subjected to the olefin hydration process conditions. The degree of hydration of the heteropolyacid may affect the acidity of the catalyst and hence its activity. Thus, either or both of these actions of impregnation and olefin hydration process may possibly change the hydration and oxidation state of the metals in the heteropolyacids, ie the actual catalytic species under the process conditions may not retain the hydration/oxidation states of the metals in the heteropolyacids used to impregnate the support. Naturally, therefore, it is to be expected that such hydration and oxidation states may also be different in the spent catalysts after the reaction.

The carrier on which the HPA catalyst is supported is suitably a siliceous support which may be in the form of gels, extrudates, pellets or granules and may be either a natural product or can be produced synthetically. This is particularly the case with silicas which may be synthetic silicas produced eg by the flame hydrolyis of silicon tetrachloride. Examples of such silicas include those commercially sold as the Grace/Davison grades of silica especially Grace 57 and 1371 silicas (ex W R Grace), and the Aerosil grades of silicas (ex Degussa) such as those claimed and described in US-A-5086031, especially Degussa 350. The supports, especially the silica supports, suitably have the following characteristics:
Pore radius (prior to use) of 10-500 Å, preferably 30-100Å
Bulk density of 0.3-0.45 g/ml, preferably 0.38-0.42 g/ml
Pore volume (water) of 0.90-1.25 ml/g, preferably 0.95-1.20 ml/g
Surface area of 200-450 m²/g, preferably 250-350 m²/g
Average particle size of 0.1-3.5 mm, preferably 0.5-2 mm.

During the impregnation of the support with the heteropolyacid catalyst in order to make the catalyst composition, it is common practice to either immerse the support into a solution such as eg an aqueous solution of the catalyst. Other impregnation techniques such as the incipient wetness technique can also be used.

The wet supported catalyst thus formed is then suitably placed in an oven at elevated temperature for several hours to dry, after which time it is allowed to cool to ambient temperature in a desiccator. The weight of the catalyst on drying, the weight of the support used and the weight of the catalyst on support is obtained by deducting the latter from the former from which the catalyst loading in g/litre can be determined. This catalyst (measured by weight) is then used in the olefin hydration process.

The catalyst loading on the support is suitably in the range from 40 to 200 g/litre, preferably from 50 to 180 g/litre.

The catalyst composition so formed may also be further modified by the addition of other acidic components thereto in order to optimise the catalytic activity thereof.

The olefin hydration process is suitably carried out using the following reaction conditions:
a. the mole ratio of water to mixed olefin passing through the reactor is suitably in the range from 0.2-1.5, preferably 0.3-0.6
b. the gas hourly space velocity (GHSV) of the water/olefin mixture is suitably from 750-5000 h⁻¹ of the catalyst composition, preferably from 1000-2000 h⁻¹ ofthe catalyst composition.
c. the catalyst loading is from 50-200 g/litre based on the weight of the support, preferably from 60-150 g/litre.

The olefin hydration reaction is carried out at a temperature from 215-225° C. Within this temperature range, the hydration of the mixed olefin to the corresponding alcohols is preferably carried out at a temperature of about 220°C.

The mixed olefins to be hydrated may be formed by admixing pure ethylene with pure propylene or may be obtained as a mixture of olefins from eg a refinery process such as from a fluid catalytic cracking process and which comprises a mixture of C2 and C3 saturated and unsaturated hydrocarbons. The hydration process is carried out in the vapour phase, ie both the olefin and water are in the vapour phase over the catalyst composition, apart from a small proportion of each gaseous reactant which dissolves in the catalyst composition. The hydration reaction is believed to occur between such dissolved reactants. Ethers corresponding to the olefin may formed in small amounts as by-products during the reaction.

The hydration reaction is carried out by placing the catalyst composition in a reactor, sealing the reactor and then heating the catalyst composition to the reaction temperature. The catalyst composition is heated to a temperature from 215 to 225°C, eg 220°C. When the catalyst composition has attained the desired temperature a charge of the olefins and water in the vapour state is passed through the reactor. The mole ratio of water to olefins passing through the reactor is suitably in the range from 0.2 to 1.5, preferably from 0.25 to 1.0, more preferably from 0.3 to 0.6. The space velocity of water vapour/olefins mixture passing through the reactor is subject to slight variations depending upon the relative concentrations of ethylene and propylene in the feedstock.

The hydration reaction is carried out at a pressure ranging from 1000 to 9000 KPa. Within this range, the olefin mixture is hydrated at a pressure from 2000 to 7000 KPa.

The activity of the catalyst system was measured by monitoring the total amount of alcohol, ether and unreacted olefins produced over a one-hour period at standard test conditions (specified in the Examples below).

Alcohols and ether production was measured by gas chromatography (see below), whereas unreacted olefins were metered using a wet-type positive displacement flow meter.

The present invention is further illustrated with reference to the following

### Examples:

### Examples:

### A. Preparation of the Catalyst:

Pelletised silica support (Degussa 350) was crushed to an average particle size of 0.5-2.0 mm. Silicotungstic acid (H₄SiW₁₂O₄₀.26H₂O, 39.9 g, ex Japan New Metals) was dissolved in de-ionised water (200 ml). A portion of the crushed silica (35.9 g) was soaked in the aqueous heteropolyacid solution for 24 hours to impregnate the silica with the acid. Excess acid was removed by decantation and the acid impregnated silica catalyst dried at 120°C in air overnight. The catalyst was then allowed to cool in a desiccator. The heteropolyacid loading on the final cooled catalyst was calculated to be 62g/litre.

### B. Preparation ofthe Catalyst:

The above process of (A) was repeated except that silicotungstic acid (49.7 g) was dissolved in de-ionised water (250 ml) and a portion of the crushed silica (46.6 g) was impregnated with the aqueous heteropolyacid solution and processed as in (A) above. The heteropolyacid loading on the final cooled catalyst was calculated to be 83 g/litre in this case.

### C. Phosphoric acid catalysts: (Comparative Tests not according to the invention)

The comparative tests for co-hydration with phosphoric acid catalysts were performed using an orthophosphoric/Degussa 350 silica catalyst summarised in in Experiments 1,2 and 3 below in which the orthophosphoric acid loading was 181 g/l.

### D. Catalyst testing:

A portion of the catalyst (20 ml) was charged to a copper-lined stainless steel microreactor. The catalyst was heated to 200 or 220°C (at the rate of 2°C/minute) in flowing nitrogen (400 ml/minute) at 40 bar(g) (4100 KPa) pressure. A de-ionised water flow (6.0 ml/hour) was introduced into the reactor and the catalyst was treated in steam/nitrogen overnight. Care was taken to ensure that the water was pre-heated prior to the introduction into the catalyst bed. Liquid water was not allowed to condense over the catalysts.

A flow of ethylene (ca. 380 ml/minute) was introduced into the reactor and the nitrogen flow was removed. A flow of propylene (ca. 1.0 ml/minute) was introduced into the reactor. As propylene was added to the ethylene/nitrogen/water mixture the nitrogen flow was gradually decreased and the propylene flow was graually increased. The reaction conditions were altered as shown in the results Tables below and the ethanol and isopropyl alcohol productivity was monitored.

### Examples:

The results of these Examples are summarised in Table 1 below in which STY is g/l/hr:

**TABLE 1**

| Ex No. | C₃H₆ ) | Water | C₂H₄ | HPA loading | Temp | Press | Space-Time Yield | | |
|---|---|---|---|---|---|---|---|---|---|
| | (ml/min) | | | (g/l) | (°C) | (KPa) | EtOH | IPA | Ratio |
| 1 | 138.5 | 124.4 | 250 | 62 | 220 | 4000 | 15 | 22 | 0.68 |
| 2 | 185.6 | 124.4 | 380 | 62 | 220 | 4000 | 22 | 50 | 0.44 |
| 3 | 185.6 | 103.7 | 380 | 62 | 220 | 5000 | 19 | 27 | 0.70 |
| 4 | 138.5 | 103.7 | 250 | 83 | 220 | 4000 | 19 | 18 | 1.06 |
| 5 | 185.6 | 103.7 | 380 | 83 | 220 | 4000 | 15 | 20 | 0.75 |
| 6 | 138.5 | 124.4 | 380 | 83 | 220 | 4000 | 32 | 19 | 1.68 |
| 7 | 138.5 | 103.7 | 380 | 83 | 220 | 5000 | 34 | 46 | 0.74 |
| 8 | 138.5 | 124.4 | 250 | 83 | 220 | 5000 | 30 | 63 | 0.48 |

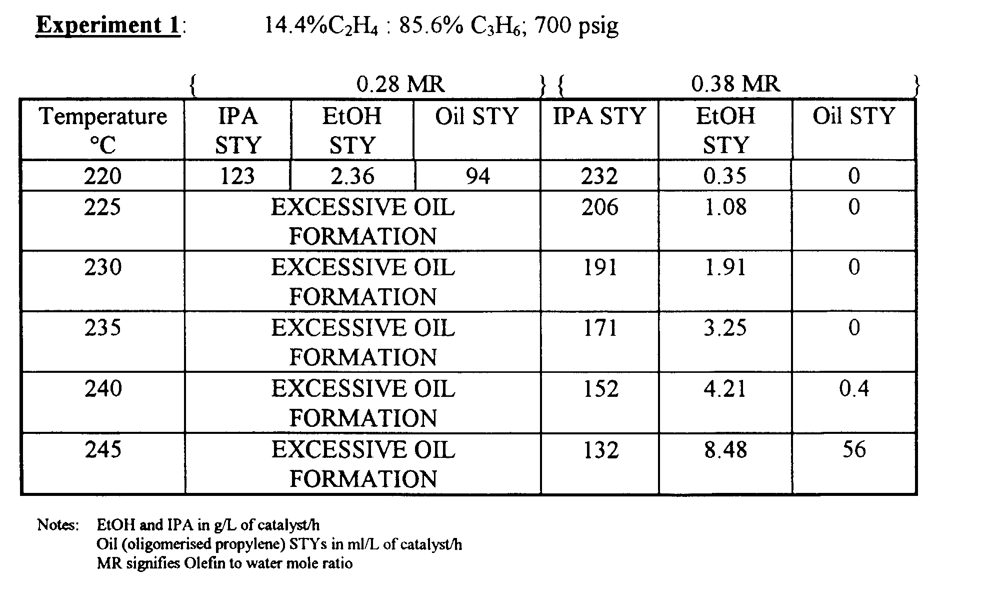

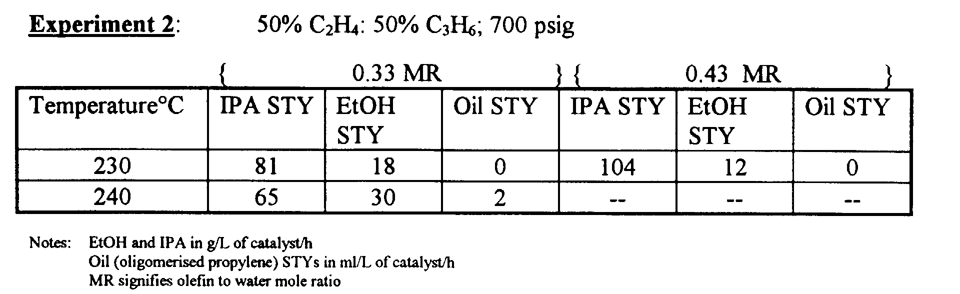

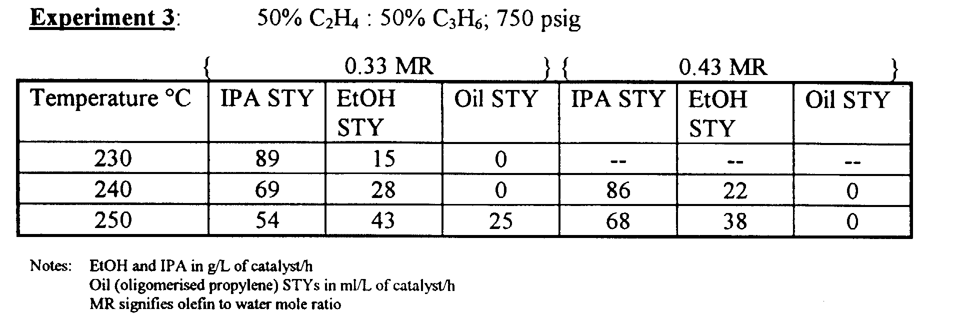

The phosphoric acid data illustrates that the catalyst cannot be used at high propylene/ethylene ratios due to excessive oil formation (expt 1). Better performance could only be only be obtained at 50:50 propylene/ethylene ratios. The overall productivity was inadequate for operation of a commercially viable process.

The HPA catalyst was more active at lower temperatures and can be used at higher propylene/ethylene ratios without excessive oil formation. Although the process was not optimised the model developed from the data predicts that better perfomance for co-hydration is possible with HPA/silica.

## Claims

1. A process for co-producing ethanol and isopropanol by hydrating a mixture of ethylene and propylene in the vapour phase in the presence of a catalyst composition comprising a heteropolyacid catalyst supported on a siliceous support, characterised in that the hydration reaction of the mixed olefin feed is carried out under the following conditions:
| | |
|---|---|
| Reaction Temp (°C) | 140-270 |
| Reaction Pressure (KPa) | 1000-9000 |
| C₂H₄:C₃H₆ mole ratio | 0.3-4.0 |
| Olefin : Water mole ratio | 0.2-1.5 |
| GHSV-Olefin/Water mix | 750-5000 |
| HPA Loading (g/l)* | 50-200 |

2. A process as claimed in claim 1 wherein the hydration reaction of the mixed nlefin feed in carried out under the following conditions:
| | |
|---|---|
| Reaction Temp (°C) | 170-250 |
| Reaction Pressure (KPa) | 2000-7000 |
| C₂H₄:C₃H₆ mole ratio | 1.0-3.0 |
| Olefin : Water mole ratio | 0.3-0.6 |
| GHSV-Olefin/Water mix | 1000-2000 |
| HPA Loading (g/l)* | 60-150 |
| | |
|---|---|
| *on the silica support | |

3. A process of claim 1 or claim 2, wherein the siliceous support which may be in the form of gels, extrudates, pellets or granules and may be either a natural product or can be produced synthetically.

4. A process as claimed in any preceding claim wherein the silicas are synthetic silicas produced by the flame hydrolyis of silicon tetrachloride.

5. A process as claimed in any preceding claim wherein the siliceous support has the following characteristics:
Pore radius (prior to use) of 10-500 Å,
Bulk density of 0.3-0.45 g/ml,
Pore volume (water) of 0.90-1.25 ml/g,
Surface area of 200-450 m²/g,
Average particle size of 0.1-3.5 mm.

6. A process as claimed in any preceding claim wherein:
a. the mole ratio of water to mixed olefin passing through the reactor is suitably in the range from 0.2-1.5,
b. the gas hourly space velocity (GHSV) of the water/olefin mixture is suitably from 750-5000 h⁻¹ of the catalyst composition, and
c. the catalyst loading is from 50-200 g/litre based on the weight of the support.
